# EUROPÄISCHE PATENTSCHRIFT

(11) **EP 0 460 544 B1**
(45) Veröffentlichungstag und Bekanntmachung des Hinweises auf die Patenterteilung: **09.08.1995**
(21) Anmeldenummer: 91108931.6
(22) Anmeldetag: 31.05.1991
(51) Int. Cl.: C07C 309/46, C07C 309/48, C07C 309/40, C07C 303/22, C07C 309/61

(54) **Verfahren zur Herstellung von sulfonierten Anthranilsäuren**
Process for the preparation of sulphonated anthranilic acids
Procédé pour la préparation d'acides anthraniliques sulphonés

(30) Priorität: 07.06.1990 DE 4018245
(43) Veröffentlichungstag der Anmeldung: 11.12.1991
(73) Patentinhaber: BASF Aktiengesellschaft, 67063 Ludwigshafen (DE)
(72) Erfinder: Patsch, Manfred, Dr., W-6706 Wachenheim (DE); Pandl, Klaus, Dr., W-6700 Ludwigshafen (DE); Dupuis, Jacques, Dr., W-6700 Ludwigshafen (DE); Hagen, Helmut, Dr., W-6710 Frankenthal (DE)

(56) Entgegenhaltungen:
- DE-A- 3 501 754
- DE-C- 138 188
- DE-C- 392 460
- FR-A- 1 380 216
- CHEMICAL ABSTRACTS Band 88, Nr. 13, 27. März 1978, Seite 486, Spalte links, Zusammenfassung Nr. 89276g, Columbus, Ohio, US; H. ASAKURA et al.: "Synthesis of some nitrotoluenesulfonic acids." & Nippon Kagaku Kaishi 1977, Band II, Seiten 1694-1697
- idem
- (BADISCHE ANILIN & SODA FABRIK AG)
- RECUEIL DES TRAVAUX CHIMIQUES DES PAYS-BAS ET DE LA BELGIQUE Band 24, Nr. 3,4, 1905, Seiten 194-208; M.M. HOLLEMAN:"L'action du cyanure de potassium sur le sel de potassium de l'acide métanitrobenzènesulfonique."
- CHEMISTRY AND INDUSTRY Band 46, 8. Oktober 1966,Seiten 1722,1723; P.S. RAHAKRISHNA MURTI et al.: "Oxidation of Tuluene and some of its derivatives by VV"

## Beschreibung

Die vorliegende Erfindung betrifft ein neues Verfahren zur Herstellung von in Ringposition 4 sulfonierten Anthranilsäuren durch Sulfonierung von ortho-Nitrotoluolen mit Chlorsulfonsäure oder Oleum, anschließender Oxidation der Methylgruppe und schließlich Reduktion der Nitrogruppe.

Es ist bekannt, 4-Methyl-3-nitrobenzolsulfonsäure durch Behandlung mit Natronlauge in Gegenwart von Kupfer-, Mangan- und Eisenoxiden in 4-Hydroxysulfonylanthranilsäure überzuführen (Chem. Abstr., Band 81, 65171 g, 1974).

Aus der CS-A-146 603 ist weiterhin bekannt, 4-Methyl-3-nitrobenzolsulfonsäure zunächst in der Hitze, unter gleichzeitiger Bestrahlung mit UV-Licht, mit Natron- oder Kalilauge zu behandeln. Die dabei entstehende Nitrobenzolsulfonsäure wird dann mittels Zink zur entsprechenden Aminoverbindung reduziert.

Bei den genannten Verfahren wird jedoch das Zielprodukt nur in unbefriedigenden Ausbeuten und begleitet von zahlreichen Nebenprodukten erhalten.

Aus Rec. trav. chim. Pays Bas, Bd. XXIV, Nr. 3 und 4, Seiten 194 bis 208, 1905, ist weiterhin ein Verfahren bekannt, in dem in Ringposition 4 sulfoniertes o-Nitrotoluol aus o-Nitrotoluol und Oleum hergestellt wird. Die sulfonierte Verbindung wird mit Salpetersäure zur Carbonsäure oxidiert und schließlich die Nitrozur Aminogruppe reduziert.

Aufgabe der vorliegenden Erfindung war es deshalb, ein neues Verfahren zur Herstellung von sulfonierten Anthranilsäuren bereitzustellen, das die obengenannten Nachteile nicht mehr aufweist. Außerdem sollte im neuen Verfahren direkt von den entsprechenden ortho-Nitrotoluolen ausgegangen werden, denn bei den bekannten Methoden wird immer eine Nitrotoluolsulfonsäure als Ausgangsprodukt verwendet, die separat hergestellt werden muß.

Es wurde nun gefunden, daß die Herstellung von Anthranilsäuren der Formel I 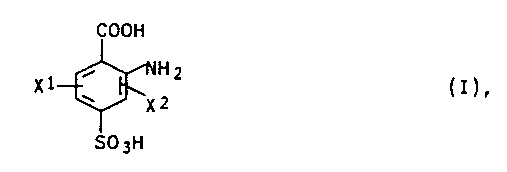
in der X¹ und X² gleich oder verschieden sind und unabhängig voneinander jeweils Wasserstoff, Halogen oder Amino bedeuten, vorteilhaft gelingt, wenn man
a) in einem 1. Schritt ein Nitrotoluol der Formel II 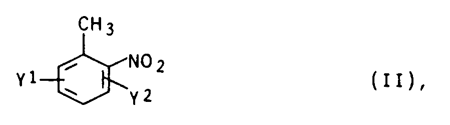 in der Y¹ und Y² gleich oder verschieden sind und unabhängig voneinander jeweils Wasserstoff, Halogen oder Nitro bedeuten, bei einer Temperatur von 40 bis 150°C mit Chlorsulfonsäure oder 10 bis 75 gew.-%igem Oleum behandelt, das Reaktionsgemisch, in dem sich die Nitrotoluolsulfonsäure der Formel III befindet 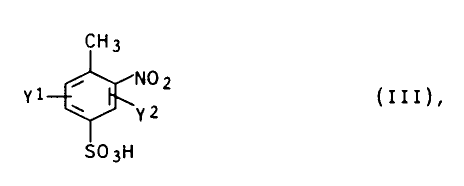 in der Y¹ und Y² jeweils die obengenannte Bedeutung besitzen,
b) in einem 2. Schritt direkt mit Wasser verdünnt und bei einer Temperatur von 130 bis 170°C mit Salpetersäure in Gegenwart einer Vanadium(V)verbindung behandelt und die resultierende Nitrobenzoesäure der Formel IV 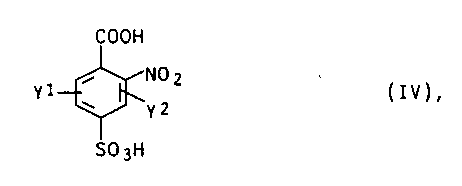 in der Y¹ und Y² jeweils die obengenannte Bedeutung besitzen,
c) in einem 3. Schritt reduziert.

Das erfindungsgemäße Verfahren wird zweckmäßig so vorgenommen, daß man das Nitrotoluol der Formel II vorlegt und unter Rühren bei einer Temperatur von 40 bis 150°C das Sulfonierungsreagenz, nämlich Chlorsulfonsäure oder 10 bis 75 gew.-%iges Oleum langsam zugibt. Dann wird bei einer Temperatur von 40 bis 150°C, bei Verwendung von Chlorsulfonsäure vorzugsweise 100 bis 150°C, und bei Verwendung von 10 bis 75 gew.-%igem Oleum vorzugsweise 40 bis 100°C, für ca. 0,5 bis 5 Stunden nachgerührt.

Je Mol Nitrotoluol II kommen dabei in der Regel 1 bis 1,3 Mol an Sulfonierungsreagenz zur Anwendung. Die Behandlung des Nitrotoluols II mit 10 bis 75 gew.-%igen Oleum, insbesondere 20 bis 30 gew.-%igem Oleum ist dabei bevorzugt.

Nach beendeter Sulfonierung wird das resultierende Reaktionsgemisch, in dem sich die Nitrotoluolsulfonsäure III befindet, direkt mit Wasser verdünnt, d.h. eine Zwischenisolierung der Nitrotoluolsulfonsäure findet nicht statt.

Dabei ist diejenige Verfahrensweise bevorzugt, bei der man soviel Wasser zugibt, daß im Reaktionsgemisch eine 60 bis 85 gew.-%ige wäßrige Schwefelsäure vorliegt.

Zu dieser Reaktionsmischung wird anschließend die Vanadium(V)verbindung gegeben. Vorzugsweise gibt man dabei Vanadiumpentoxid zu, das unter den Reaktionsbedingungen dann in Vanadium(V)sulfat übergeführt wird.

Daran anschließend erfolgt die Zugabe von Salpetersäure, wobei üblicherweise 30 bis 100 gew.-%ige Salpetersäure verwendet wird. Die Behandlung mit Salpetersäure wird bei einer Temperatur von 130 bis 170°C, vorzugsweise 145 bis 160°C und insbesondere 150 bis 160°C, vorgenommen. Während der Behandlung kann gleichzeitig verdünnte Salpetersäure (ca. 30 Gew.-%) aus dem Reaktionsgemisch abdestilliert werden.

Je Mol Nitrotoluol II kommen in der Regel 0,01 bis 0,1 Moläquivalent, vorzugsweise 0,03 bis 0,06 Moläquivalent, an Vanadium(V)verbindung und 1,2 bis 6 Mol Salpetersäure zur Anwendung.

Nach beendeter Umsetzung, die im allgemeinen 6 bis 10 Stunden in Anspruch nimmt, erfolgt die Isolierung der Nitrobenzoesäure IV. Dazu wird das Reaktionsgemisch abgekühlt und üblicherweise mit 100 bis 200 Gew.-% Wasser, bezogen auf das Reaktionsgemisch, und daran anschließend mit wäßriger Alkalihydroxidlösung, z.B. Natronlauge oder Kalilauge, versetzt bis ein pH-Wert von ca. 0 bis 1 erreicht ist.

Nach Kühlen auf ca. 0°C fällt die Nitrobenzoesäure IV als Niederschlag an, der abgetrennt und in der Regel ohne weitere Reinigung der Hydrierungsreaktion unterworfen wird.

Es ist auch mögich, auf die Isolierung der Nitrobenzoesäure IV zu verzichten. In diesem Fall nimmt man die Reduktion der Nitrobenzoesäure IV direkt in dem Reaktionsgemisch vor, wie es bei der Oxidation anfällt.

Die Reduktion der Nitrobenzoesäure IV erfolgt nach an sich bekannten Methoden, wie sie beispielsweise in Houben-Weyl, "Methoden der Organischen Chemie", Band 11/1, S. 360 ff, beschrieben sind.

Vorzugsweise führt man eine katalytische Reduktion mittels Wasserstoff durch, wobei man Palladium oder insbesondere Raney-Nickel als Katalysator verwendet. Palladium wird dabei im allgemeinen in Form eines Trägerkatalysators angewandt, wobei als Träger insbesondere Kohle zu nennen ist.

Die katalytische Hydrierung führt man im allgemeinen in wäßrigem Medium bei einer Temperatur von 20 bis 60°C und einem Wasserstoff-Druck von 1,0 bis 6,0 bar durch. Der pH-Wert liegt üblicherweise bei 4 bis 11. Nachdem die Wasserstoffaufnahme beendet ist, wird in der Regel vom Katalysator abfiltriert und das Filtrat angesäuert (pH-Wert von -0,5 bis +3,0, vorzugsweise 0 bis 1). Die als Niederschlag anfallende Anthranilsäure I wird dann abgetrennt.

Eine weitere Möglichkeit der Isolierung besteht in der direkten Sprühtrocknung der vom Hydrierkatalysator abfiltrierten Reduktionslösung, wobei je nach pH-Wert der Lösung das Zielprodukt entweder in Form der freien Säure oder in Salzform (z.B. als Alkalisalz) erhalten wird.

Es hat sich gezeigt, daß bei der katalytischen Hydrierung mit Raney-Nickel als Katalysator, die Reaktionsgeschwindigkeit durch Zugabe einer aliphatischen Mono- oder Dicarbonsäure, die 2 bis 10 Kohlenstoffatome aufweist, z.B. Essigsäure, Propionsäure oder Adipinsäure, zum Raktionsgemisch erhöht werden kann. Im allgemeinen kommen dabei 5 bis 50 g Carbonsäure, bezogen auf 1 Mol Nitrotoluol II, zur Anwendung.

Das erfindungsgemäße Verfahren kann sowohl in kontinuierlicher als auch in diskontinuierlicher Arbeitsweise vorgenommen werden. Es liefert die Zielprodukte in guter Ausbeute und hoher Reinheit. Ein weiterer Vorteil des neuen Verfahrens liegt darin, daß man, wie eingangs schon erwähnt, direkt von den Nitrotoluolen der Formel II ausgehen kann und die aus diesen Verbindungen gewonnenen Sulfonsäuren nicht zwischenisolieren muß.

Die mittels des erfindungsgemäßen Verfahrens erhältlichen sulfonierten Anthranilsäuren I sind wertvolle Zwischenprodukte für die Herstellung von Farbstoffen, z.B. von Kupferformazanen, wie in der EP-A-315 046 beschrieben.

Die folgenden Beispiele sollen die Erfindung näher erläutern.

### Beispiel 1

### Herstellung von 2-Nitro-4-hydroxysulfonylbenzoesäure

548 g (4 mol) ortho-Nitrotoluol wurden vorgelegt. Dazu wurden bei einer Temperatur von ca. 70°C 1350 g 24 gew.-%iges Oleum innerhalb von 2 Stunden zugetropft. Dann wurde 30 Minuten bei dieser Temperatur nachgerührt, wonach 420 ml Wasser und 40 g Vanadiumpentoxid zum Reaktionsgemisch gegeben wurden. Die Reaktionsmischung wurde dann auf 155°C erwärmt und innerhalb von 8,5 Stunden wurden 2550 ml 65 gew.-%ige Salpetersäure zudosiert. Gleichzeitig wurden dabei 2,5 l ca. 30 gew.-%ige Salpetersäure aus dem Reaktionsgemisch abdestilliert.

Danach wurde die Reaktionslösung mit 3205 ml Wasser und 801 g 50 gew.-%iger Natronlauge versetzt (pH des Reaktionsgemisches ca. 0) und innerhalb von 5 Stunden auf 0°C abgekühlt. Die als Niederschlag anfallende 2-Nitro-4-hydroxysulfonylbenzoesäure wurde abgesaugt, gewaschen und getrocknet. Ausbeute 76 %.

### Beispiel 2

### Herstellung von 4-Hydroxysulfonylanthranilsäure

494 g 2-Nitro-4-hydroxysulfonylbenzoesäure wurden in 3,5 l Wasser bei einem pH-Wert von 6,0 gelöst und mit 4 g eines Palladiumkatalysators (10 Gew.-% Palladium auf Kohle) mit Wasserstoff (Druck: 3 bar) bei einer Temperatur von 40°C hydriert. Nach Ende der Wasserstoffaufnahme wurde der Katalysator abfiltriert.

Das Filtrat wurde mit Salzsäure auf einen pH-Wert von 0 gestellt und der gebildete Niederschlag durch Filtration isoliert. Man erhielt 410 g 4-Hydroxysulfonylanthranilsäure.

### Beispiel 3

### Herstellung von 4-Hydroxysulfonylanthranilsäure

Es wurde analog Beispiel 2 verfahren, jedoch wurde das Filtrat sprühgetrocknet. Man erhielt 440 g 4-Hydroxysulfonylanthranilsäure.

### Beispiel 4

### Herstellung von 4-Hydroxysulfonylanthranilsäure

494 g 2-Nitro-4-hydroxysulfonylbenzoesäure wurden in 2,5 l Wasser unter Zusatz von 50 g Propionsäure bei einem pH-Wert von 4,0 gelöst und mit 10 g Raney-Nickel als Katalysator mit Wasserstoff (Druck: 3 bar) bei einer Temperatur von 40°C hydriert. Nach Ende der Wasserstoffaufnahme wurde der Katalysator abfiltriert.

Das Filtrat wurde mit Salzsäure auf einen pH-Wert von 0 gestellt und der gebildete Niederschlag durch Filtration isoliert. Man erhielt 400 g 4-Hydroxysulfonylanthranilsäure.

### Beispiel 5

### Herstellung von 4-Hydroxysulfonylanthranilsäure

Es wurde analog Beispiel 4 verfahren, jedoch wurde das Filtrat sprühgetrocknet. Man erhielt 440 g 4-Hydroxysulfonylanthranilsäure.

## Patentansprüche

1. Verfahren zur Herstellung von Anthranilsäuren der Formel I 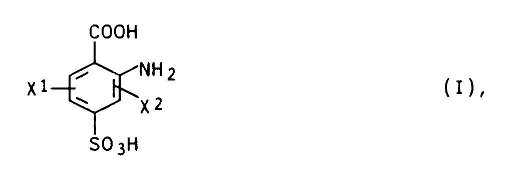 in der X¹ und X² gleich oder verschieden sind und unabhängig voneinander jeweils Wasserstoff, Halogen oder Amino bedeuten, dadurch gekennzeichnet, daß man
a) in einem 1. Schritt ein Nitrotoluol der Formel II 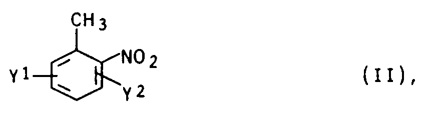 in der Y¹ und Y² gleich oder verschieden sind und unabhängig voneinander jeweils Wasserstoff, Halogen oder Nitro bedeuten, bei einer Temperatur von 40 bis 150°C mit Chlorsulfonsäure oder 10 bis 75 gew.-%igem Oleum behandelt, das Reaktionsgemisch, in dem sich die Nitrotoluolsulfonsäure der Formel III befindet 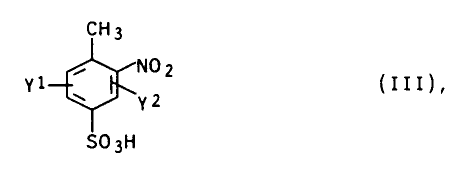 in der Y¹ und Y² jeweils die obengenannte Bedeutung besitzen,
b) in einem 2. Schritt direkt mit Wasser verdünnt und bei einer Temperatur von 130 bis 170°C mit Salpetersäure in Gegenwart einer Vanadium(V)verbindung behandelt und die resultierende Nitrobenzoesäure der Formel IV 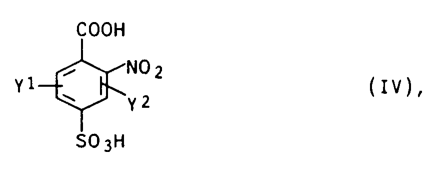 in der Y¹ und Y² jeweils die obengenannte Bedeutung besitzen,
c) in einem 3. Schritt reduziert.

2. Verfahren nach Anspruch 1, dadurch gekennzeichnet, daß X¹ und X² jeweils Wasserstoff bedeuten.

3. Verfahren nach Anspruch 1, dadurch gekennzeichnet, daß man in Stufe a) die Behandlung des Nitrotoluols der Formel II mit 10 bis 75 gew.-%igem Oleum vornimmt.

## Claims

1. A process for preparing anthranilic acids of the formula I 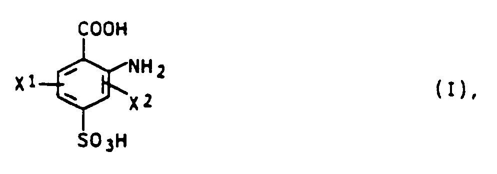 where X¹ and X² are identical or different and each is independently of the other hydrogen, halogen or amino, characterized in that
a) in a 1st step a nitrotoluene of the formula II 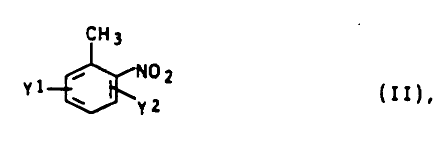 where Y¹ and Y² are identical or different and each is independently of the other hydrogen, halogen or nitro, is treated at a temperature from 40 to 150°C with chlorosulfonic acid or with from 10 to 75% strength by weight oleum, the reaction mixture which contains the nitrotoluenesulfonic acid of the formula III 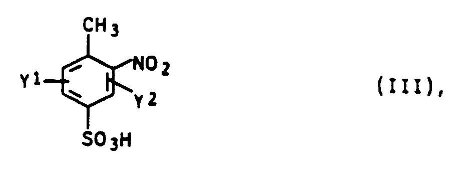 where Y¹ and Y² are each as defined above,
b) is in a 2nd step directly diluted with water and treated at a temperature from 130 to 170°C with nitric acid in the presence of a vanadium(V) compound, and the resulting nitrobenzoic acid of the formula IV 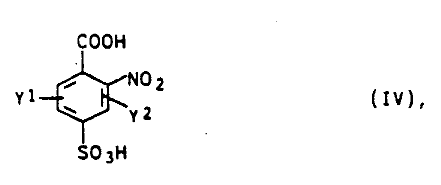 where Y¹ and Y² are each as defined above,
c) is reduced in a 3rd step.

2. A process as claimed in claim 1, characterized in that X¹ and X² are each hydrogen.

3. A process as claimed in claim 1, characterized in that in stage a) the treatment of the nitrotoluene of the formula II is carried out with from 10 to 75% strength by weight oleum.

## Revendications

1. Procédé de préparation d'acides anthraniliques de formule I 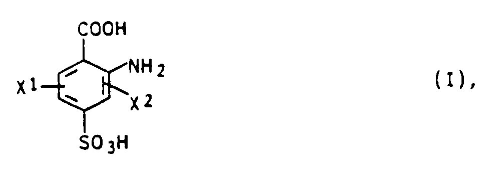 dans laquelle X¹ et X² sont identiques ou différents et représentent chacun, indépendamment l'un de l'autre, un atome d'hydrogène, un atome d'halogéne ou un groupement amino, caractérisé en ce que
a) dans une 1ère étape, on traite un nitrotoluène de formule II 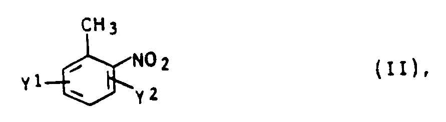 dans laquelle Y¹ et Y² sont identiques ou différents et représentent chacun, indépendamment l'un de l'autre, un atome d'hydrogène, un atome d'halogène ou un groupement nitro, à une température de 40 a 150°C, par de l'acide chlorosulfonique ou de l'oléum à 10-75% en poids,
b) dans une 2ème étape, on dilue directement à l'eau le mélange réactionnel, dans lequel se trouve l'acide nitrotoluènesulfonique de formule III 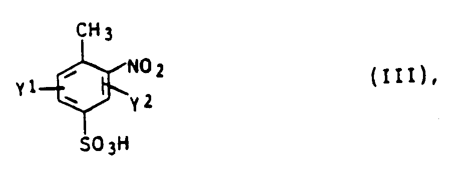 dans laquelle Y¹ et Y² ont chacun la signification donnée ci-dessus, et on le traite, à une température de 130 à 170°C, par de l'acide nitrique en présence d'un composé de vanadium(V) et
c) dans une 3ème étape, on réduit l'acide nitrobenzoïque résultant de formule IV 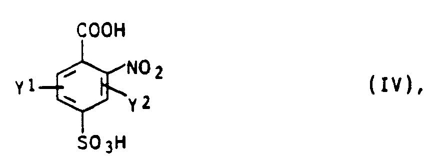 dans laquelle Y¹ et Y² ont chacun la signification donnée ci-dessus.

2. Procédé selon la revendication 1, caractérisé en ce que X¹ et X² représentent chacun un atome d'hydrogène.

3. Procédé selon la revendication 1, caractérisé en ce que, dans l'étape a), on procède au traitement du nitrotoluène de formule II par de l'oléum a 10-75% en poids.
